**Europäisches Patentamt**

**European Patent Office**

**Office européen des brevets**

(11) Numéro de publication : **0 499 512 B1**

(12) **FASCICULE DE BREVET EUROPEEN**

(45) Date de publication du fascicule du brevet :
**16.02.94 Bulletin 94/07**

(51) Int. Cl.⁵ : **C07C 19/045,** C07C 17/156

(21) Numéro de dépôt : **92400307.2**

(22) Date de dépôt : **06.02.92**

54 **Procédé de synthèse du 1,2 dichloroéthane par oxychloration de l'éthylène au moyen du tétrachlorure de carbone.**

(30) Priorité : **15.02.91 FR 9101824**

(43) Date de publication de la demande :
**19.08.92 Bulletin 92/34**

(45) Mention de la délivrance du brevet :
**16.02.94 Bulletin 94/07**

(84) Etats contractants désignés :
**AT BE CH DE DK ES FR GB GR IT LI LU NL PT SE**

56 Documents cités :
**GB-A- 1 351 700**

73 Titulaire : **ELF ATOCHEM S.A.**
**4 & 8, Cours Michelet La Défense 10**
**F-92800 Puteaux (FR)**

72 Inventeur : **Correia, Yves**
**Les Lauzière**
**F-04160 Château Arnoux (FR)**
Inventeur : **Chastel, Gérard**
**Villa Heurtbise, Paresous**
**F-04200 Sisteron (FR)**

## Description

La présente invention concerne un procédé de synthèse du 1,2 dichloroéthane par oxychloration de l'éthylène au moyen du tétrachlorure de carbone. Par chloration de l'éthylène, on obtient le 1,2-dichloroéthane (D12) qui par simple pyrolyse donne du chlorure de vinyle (CVM) et de l'acide chlorhydrique (HCl). HCl est recyclé pour produire du D12 par la réaction d'oxychloration suivante :

$$(1) \qquad 2\ C_2H_4 + O_2 + 4\ HCl \rightarrow 2\ C_2H_4Cl_2 + 2\ H_2O$$

Cette réaction d'oxychloration permet de valoriser le chlore contenu dans HCl.

Ces procédés sont décrits dans ULLMANN'S ENCYCLOPEDIA OF CHEMICAL INDUSTRY, 5ème édition, vol. A 6 , pages 263 - 270 et 283 -291.

Un but de l'invention est de valoriser le chlore contenu dans le $CCl_4$. En effet, au cours de la synthèse des chlorométhanes on produit inévitablement du $CCl_4$ en trop grande quantité. La demanderesse a ainsi découvert qu'on pouvait l'utiliser comme source de chlore dans une réaction d'oxychloration de l'éthylène, c'est-à-dire qu'on peut faire passer un mélange de $CCl_4$, $C_2H_4$ et $O_2$ (ou air) sur un catalyseur d'oxychloration et produire du D12 et en moindre quantité ses homologues supérieurs tels que le tri et tétrachloroéthane.

Dans le brevet FR 2260551, on a décrit l'oxychloration de résidus chlorés lourds, c'est-à-dire, la réaction entre ces résidus de l'éthylène et de l'oxygène qui permet de produire du perchloroéthylène, du trichloroéthane, du tétrachloroéthane et du D12.

Dans la demande de brevet ZA 71-5781 publiée le 27 mars 1972, il est décrit un procédé d'oxychloration de l'éthylène pour préparer un mélange de perchloroéthylène et trichloroéthylène.

Selon cet art antérieur, l'éthylène, l'oxygène et le chlore passent sur un catalyseur en lit fluidisé à base de chlorure de cuivre sur un support poreux et à une température entre 410° et 440°C. La réaction se fait en présence de tétrachlorure de carbone provenant du recyclage de produits de sortie du lit fluidisé. Les produits sortant du lit fluidisé sont refroidis pour condenser du perchloroéthylène, trichloroéthylène et du tétrachlorure de carbone. Ce dernier ($CCl_4$) est mélangé aux gaz d'alimentation ($C_2H_4$, $Cl_2$, air) et ainsi recyclé au lit fluidisé. Le perchloroéthylène et trichloroéthylène représentent au moins 50 % en moles des produits chlorés en sortie du lit fluidisé.. La quantité de $CCl_4$ est constante, il ne s'accumule pas. Sa concentration ne dépasse pas 10 % molaire par rapport aux composés organiques à l'entrée du lit fluidisé, ce qui correspond à quelques % par rapport à la totalité des gaz à l'entrée. La quantité de chlore sous forme $Cl_2$ représente entre 40 et 75 % des réactifs chlorés à l'entrée du lit fluidisé, environ 2 à 8 % est sous forme HCl et le reste est des chlorés organiques (et parmi eux le $CCl_4$). Le rapport atomique oxygène sur carbone à l'entrée du lit fluidisé est compris entre 0,85 et 2. Le bilan de cette réaction est donc la production de perchloroéthylène et trichloroéthylène en consommant de l'éthylène, du chlore ($Cl_2$) et de l'oxygène. La présente invention est très différente puisqu'elle consiste à produire du D12, et en moindre quantité ses homologues supérieurs, en consommant de l'éthylène, du $CCl_4$ et de l'oxygène. Le but de l'invention est donc de faire la réaction :

$$(2) \qquad CCl_4 + 2\ C_2H_4 + O_2 \rightarrow 2\ C_2H_4Cl_2 + CO_2$$

La présente invention est un procédé de synthèse de 1,2-dichloroéthane caractérisé en ce qu'on met en contact du tétrachlorure de carbone, de l'éthylène et de l'oxygène avec un catalyseur d'oxychloration à une température suffisante pour former du 1,2-dichloroéthane puis qu'on sépare ce dernier des produits de la réaction.

La réaction est effectuée dans les conditions normales d'oxychloration en lit fixe ou en lit fluide sur tout catalyseur à base d'alumine, silice magnésie, atapulgite et contenant du cuivre, mais pouvant aussi contenir les additifs habituels, potassium, magnésium, terres rares.

Des catalyseurs qui conviennent sont décrits, par exemple, dans les brevets FR 1 555 518, FR 2 260 551, FR 2 063 365, FR 2 213 259, FR 2 141 452, EP 255 156, EP 257 561 et EP 176 432.

Ces catalyseurs sont en général constitués de chlorure de cuivre déposé sur de l'alumine de surface spécifique comprise entre 50 et 450 m²/g et de granulométrie entre 20 et 200 microns. La quantité de chlorure de cuivre exprimée en cuivre peut être comprise entre 3 et 12 % de la masse totale du catalyseur.

On peut aussi utiliser des catalyseurs constitués d'un mélange des catalyseurs précédents et d'une substance inerte telle que du sable. De tels catalyseurs sont décrits dans FR 2 242 143 et EP 377 364.

Tous ces catalyseurs sont utilisés en lit fluidisé mais on ne sortirait pas du cadre de l'invention en effectuant une catalyse en lit fixe.

La température de la réaction est comprise entre 220 et 450 °C, sachant qu'il est nécessaire d'avoir une température telle que la transformation du $CCl_4$ ne s'arrête pas au dérivé chloré oxygéné en C1. La pression utilisée est comprise entre la pression atmosphérique et les limites habituelles de ce type de procédé, c'est-à-dire, inférieur à 10 bars.

La réaction s'effectue en présente d'oxygène qui est le plus souvent sous forme d'air.

On ne sortirait pas du cadre de l'invention en ajoutant de l'acide chlorhydrique. Les proportions d'acide chlorhydrique et de tétrachlorure de carbone peuvent être quelconques, c'est-à-dire que la réaction de l'invention est comprise entre les deux équations de réaction (1) et (2).

Le débit d'air (d'oxygène) et le débit d'éthylène sont ajustés pour obtenir la conversion presque complète de l'éthylène et l'absence d'HCl en sortie. Ce réglage est connu de l'homme de métier de l'oxychloration.

Avantageusement la proportion de $CCl_4$ et d'éthylène est telle que le rapport molaire $\dfrac{CCl_4}{C_2H_4}$ est compris entre 0,4 et 1,6.

Si la réaction s'effectue en présence d'HCl, dans le rapport $\dfrac{CCl_4}{C_2H_4}$ "$CCl_4$" représente la somme du nombre de moles de $CCl_4$ et d'un quart du nombre de moles d'HCl.

Avantageusement la proportion d'oxygène et d'éthylène est telle que le rapport molaire $O_2/C_2H_4$, O2 étant exprimé en air, est compris entre 3 et 10. C'est-à-dire qu'un rapport de 5 signifie une mole d'oxygène pour une mole d'éthylène.

Selon une forme préférée de l'invention, on ajoute de la vapeur d'eau au mélange réactionnel à l'entrée du lit catalytique c'est-à-dire que le mélange réactionnel consiste en $C_2H_4$, $O_2$ (air), $CCl_4$ (avec éventuellement HCl) et $H_2O$.

La quantité d'eau peut être quelconque et peut aller jusqu'à 2,5 moles par mole de $CCl_4$ à l'entrée du lit catalytique.

A la sortie du lit catalytique, la récupération des produits se fait par les moyens connus de l'homme de métier de l'oxychloration, à savoir un refroidissement pour condenser les produits les moins volatiles et les séparer des gaz qui n'ont pas réagi, puis une ou plusieurs distillations.

La demanderesse essaye d'expliquer l'effet de l'eau en pensant que le tétrachlorure de carbone est hydrolysé selon la réaction (3) :

$$(3) \qquad CCl_4 + 2\,H_2O \rightarrow 4\,HCl + CO_2$$

puis que le HCl ainsi formé réagit selon la réaction habituelle (1) :

$$(1) \qquad 2\,C_2H_4 + O_2 + 4\,HCl \rightarrow 2\,C_2H_4Cl_2 + 2\,H_2O.$$

Dans les exemples suivants, les temps de séjour définis comme le rapport du volume du catalyseur (expansé s'il s'agit d'une oxychloration en lit fluide) au débit des réactifs à l'entrée du lit à la température du réacteur sont de ceux habituellement utilisés en oxychloration.

## EXEMPLE 1

Dans un réacteur en verre de diamètre 24 mm mais de surface 4,2412 cm² (déduction faite de la gaine de température le traversant), de hauteur 1 mètre et surmonté d'un vase d'expansion, on place 160 g d'un catalyseur silice magnésie (surface spécifique 80 m2/g) imprégné de 3,5 % de cuivre et contenant du potassium dans un rapport

$\dfrac{K}{Cu}$ atomique égal 0,6

et 2,1 % de terres rares. Ce catalyseur est décrit dans le brevet FR 2 125 748 dont le contenu est incorporé dans la présente demande. On imprègne des microbilles d'hydrogel de silice par une solution de chlorure de magnésium qu'on calcine ensuite puis on imprègne la poudre obtenue avec du chlorure de cuivre. Par un système de régulation, le lit est maintenu à 340°C et on introduit :

| | |
|---|---|
| Air | 1303,1 mmole/heure |
| $H_2O$ | 307,0 mmole/heure |
| $CCl_4$ | 194,3 mmole/heure |
| $C_2H_4$ | 152,0 mmole/heure |

La hauteur du lit ainsi fluidisé est de 77 cm et le temps de séjour de l'ordre de 12 secondes.

Les gaz sortant du réacteur, refroidis à 18°C abandonnent 22,9 g d'un liquide organique dont la composition est la suivante en % poids :

| | | |
|---|---|---|
| Dichloroéthylène | 0,17 | |
| $CHCl_3$ | 0,08 | |
| $CCl_4$ | 30,68 | |
| $C_2H_4Cl_2$ (D12) | 18,58 | |
| Trichloréthylène (Tri) | 0,74 | |
| $C_2H_3Cl_3$ As | 29,33 | AS=Assymétrique |
| Perchloréthylène (Per) | 0,93 | |
| Tétrachloréthane AS | 0,17 | |

| | | |
|---|---|---|
| Tétrachloréthane S | 15,82 | S = Symétrique |
| Pentachloréthane | 3,4 | |
| Hexachloréthane | 0,1 | |

Le gaz sortant après condensation 31,27 Nlitre contient en volume 9,2 % de $CO_2$ et 12 % d'oxygène. Il est exempt d'éthylène et saturé en solvant à la température de 18°C.

Le taux de transformation du $CCl_4$ est de 50 % et celui de l'éthylène de 99,8 %.

**EXEMPLE 2**

On utilise le même réacteur décrit dans l'exemple 1 mais il est complété par des instruments de mesure habituellement utilisés industriellement en ligne sur les gaz sortant des réacteurs d'oxychloration. On procède alors sur le même catalyseur décrit à l'exemple 1 à l'introduction des réactifs : air, acide chlorhydrique, éthylène et tétrachlorure de carbone. Les conditions opératoires, température, temps de séjour, débit d'entrée des réactifs ainsi que les débit des réactifs en excès et des produits formés sont donnés dans le tableau en annexe à l'intitulé exemple 2. Le taux de conversion du $CCl_4$ est de 47 %, celui de l'éthylène 99,8 % et celui de l'oxygène de 79,7 %.

**EXEMPLES 3 et 4**

Dans l'appareillage décrit dans l'esemple 1 et complété par les instruments analytiques mentionnés dans l'exemple 2 on procède à deux nouveaux essais dont les conditions opératoires, les débits d'entrée des réactifs et les débits de sortie des réactifs en excès et des produits formés sont donnés dans le tableau en annexe aux intitulés exemple 3 et 4.

A titre d'exemple non conforme à l'invention on procède à l'introduction sur le même catalyseur que l'exemple 1 et à la température de 340°C des débits suivants : air 1 303 mmole/H, $CCl_4$ 182 mmole/H, $H_2O$ 394 mmole/H ; le temps de séjour étant de 11 secondes on constate une conversion du $CCl_4$ de 39 %.

ANNEXE

| Exemples | 2 | 3 | 4 |
|---|---|---|---|
| Température réacteur | 338,7 | 341,5 | 300 |
| Temps séjour en sec. | 9,8 | 10,1 | 17,2 |
| Débit entrée en mmol/H | | | |
|     Air | 1 303 | 1 303 | 928,6 |
|     HCl | 575 | 0 | 0 |
|     $CCl_4$ | 50 | 233,8 | 219,6 |
|     $H_2O$ | 0 | 489 | 0 |
|     $C_2H_4$ | 304 | 304 | 165,2 |
| Débit sortie en mmol/H | | | |
|     Di+légers | 5,01 | 9,0 | 1,39 |
|     $HCCl_3$ | 1,81 | 1,91 | 0,16 |
|     $CCl_4$ | 3,42 | 128,3 | 152,22 |
|     Tri | 1,15 | 0,23 | 0 |
|     T112 | 43,9 | 6,82 | 3,36 |
|     PER | 0,31 | 0,10 | 0,036 |
|     T4S | 10,98 | 0,3 | 0,198 |
|     Penta | 0,9 | 0 | 0,015 |
|     D12 | 226 | 184,0 | 124,2 |
|     $CO_2$ ⎧ Total | 33,6 | 211,1 | 137,8 |
|          ⎩ $CCl_4$ | 23,5 | 105,5 | 67,4 |
|     CO | 3,1 | 16,7 | 6,2 |
|     $C_2H_4$ | ⩽ 0,5 | 41,7 | 4,4 |
|     $O_2$ | 55,2 | 0 | 26,25 |
|     $N_2$ (analyse) | 1 027 | 1 032 | 699 |
|     HCl | 7,15 | 6,4 | 1,75 |
| Taux transformation | | | |
|     $CCl_4$  % | 4,7 | 45,1 | 30,7 |
|     $C_2H_4$  % | 99,8 | 86,3 | 97,3 |
|     $O_2$    % | 79,7 | ≃ 100 | 86,5 |

**Revendications**

1. Procédé de synthèse de 1,2-dichloroéthane caractérisé en ce qu'on met en contact du tétrachlorure de carbone, de l'éthylène et de l'oxygène avec un catalyseur d'oxychloration à une température suffisante pour former du 1,2-dichloroéthane puis qu'on sépare ce dernier des produits de la réaction.

2. Procédé selon la revendication 1 caractérisé en ce que le catalyseur d'oxychloration est en lit fluidisé.

3. Procédé selon la revendication 1 ou 2 caractérisé en ce qu'on ajoute de l'acide chlorhydrique aux réactifs.

4. Procédé selon l'une des revendications 1 à 3 caractérisé en ce que la température de réaction est comprise entre 220 et 450°C.

5. Procédé selon l'une des revendications 1 à 4 caractérisé en ce qu'on ajoute de la vapeur d'eau au mélange réactionnel avant le catalyseur d'oxychloration.

6. Procédé selon l'une des revendications 1 à 5 caractérisé en ce que le rapport molaire tétrachlorure de carbone (y compris l'acide chlorhydrique éventuel exprimé en $CCl_4$) sur éthylène est compris entre 0,4 et 1,6.

7. Procédé selon l'une des revendications 1 à 6 caractérisé en ce que le rapport molaire $O_2/C_2H_4$ ($O_2$ exprimé en air) est compris entre 3 et 10.

**Patentansprüche**

1. Verfahren zur Herstellung von 1,2-Dichlorethan, dadurch gekennzeichnet, daß man Tetrachlorkohlenstoff, Ethylen und Sauerstoff mit einem Oxychlorierungskatalysator bei einer genügend hohen Temperatur zur Herstellung von 1,2 Dichlorethan in Berührung bringt und dann dieses letztere aus den Reaktionsprodukten abtrennt.

2. Verfahren nach Anspruch 1, dadurch gekennzeichnet, daß der Oxychlorierungskatalysator in einem Fließbett eingesetzt wird.

3. Verfahren nach Anspruch 1 oder 2, dadurch gekennzeichnet, daß man Chlorwasserstoffsäure zu den Ausgangsstoffen hinzufügt.

4. Verfahren nach einem der Ansprüche 1 bis 3, dadurch gekennzeichnet, daß die Reaktionstemperatur zwischen 220 und 450°C liegt.

5. Verfahren nach einem der Ansprüche 1 bis 4, dadurch gekennzeichnet, daß man Wasserdampf zu der Reaktionsmischung vor dem Oxychlorierungskatalysator zufügt.

6. Verfahren nach einem der Ansprüche 1 bis 5, dadurch gekennzeichnet, daß das molare Verhältnis Tetrachlorkohlenstoff (darin gegebenenfalls enthaltend Chlorwasserstoffsäure, bezogen auf $CCl_4$) zu Ethylen zwischen 0,4 und 1,6 liegt.

7. Verfahren nach einem der Ansprüche 1 bis 6, dadurch gekennzeichnet, daß das molare Verhältnis $O_2/C_2H_4$ ($O_2$ bezogen auf Luft) zwischen 3 und 10 liegt.

**Claims**

1. Process for the synthesis of 1,2-dichloroethane, characterized in that carbon tetrachloride, ethylene and oxygen are brought into contact with an oxychlorination catalyst at a temperature sufficient to form 1,2-dichloroethane, and that thereafter the latter is separated from the reaction products.

2. Process according to Claim 1, characterized in that the oxychlorination catalyst is in a fluidized bed.

3. Process according to Claim 1 or 2, characterized in that hydrochloric acid is added to the reactants.

4. Process according to one of Claims 1 to 3, characterized in that the reaction temperature is between 220 and 450°C.

5. Process according to one of Claims 1 to 4, characterized in that water vapour is added to the reaction mixture before the oxychlorination catalyst.

6. Process according to any one of Claims 1 to 5, characterized in that the molar ratio of carbon tetrachloride (including any hydrochloric acid, expressed as $CCl_4$) to ethylene is between 0.4 and 1.6.

7. Process according to one of Claims 1 to 6, characterized in that the molar ratio $O_2/C_2H_4$ ($O_2$ being expressed as air) is between 3 and 10.